# EUROPEAN PATENT APPLICATION

(11) **EP 1 880 729 A1**
(43) Date of publication of application: **23.01.2008**
(21) Application number: 06291177.1
(22) Date of filing: 20.07.2006
(51) Int. Cl.: A61K 38/17, A61P 37/06

(54) **Use of soluble CD160 to suppress immunity**

(71) Applicant: INSERM (Institut National de la Santé et de la Recherche Médicale), 75654 Paris Cedex 13 (FR)
(72) Inventor: Bensussan, Armand, 75013 Paris (FR); Boumsell, Laurence, 75015 Paris (FR); Ortonne, Nicolas, 94220 Charenton le Pont (FR)
(74) Representative: Touati, Catherine

(57) **Abstract**

Pharmaceutical composition comprising a soluble form of CD160 for treating an inflammatory condition involving an undesired immune response, such as tissue graft or organ rejection, and autoimmune diseases; in vitro method for screening an individual for the presence of an inflammatory condition such as infectious and autoimmune diseases, tissue graft and organ rejection, or the presence of a tumor or activated endothelial cells, or for monitoring therapy of an inflammatory condition such as an autoimmune disorder or a tissue or organ rejection, or a tumor during chemotherapy including treatment with an anti-angiogenic substance or antibody.

SEQUENCE LISTING

<110>  INSERM
       Bensussan, Armand
       Boumsell, Laurence

<120>  Use of soluble CD160 to suppress immunity

<130>  BEP060234

<160>  7

<170>  PatentIn version 3.3

<210>  1
<211>  21
<212>  DNA
<213>  artificial

<220>
<223>  CD160 forward primer

<400>  1
tgcaggatgc tgttggaacc c                                                21


<210>  2
<211>  22
<212>  DNA
<213>  artificial

<220>
<223>  CD160 reverse primer

<400>  2
cctgtgccct gttgcattct tg                                               22


<210>  3
<211>  24
<212>  DNA
<213>  artificial

<220>
<223>  GPI-PLD1 variant 1 forward primer

<400>  3
atggatggcg tgcctgacct ggcc                                             24


<210>  4
<211>  24
<212>  DNA
<213>  artificial

<220>
<223>  GPI-PLD1 variant 1 reverse primer

<400>  4
cagcggtggc tgcaggtcgg atgt                                             24


<210>  5
<211>  24
<212>  DNA
<213>  artificial

<220>

<223>  GPI-PLD1 variant 2 forward primer

<400>  5
gtgttggact ttaacgtgga cggc                                              24


<210>  6
<211>  24
<212>  DNA
<213>  artificial

<220>
<223>  GPI-PLD1 variant 2 reverse primer

<400>  6
cagcagaggc tgcgcgtcag atat                                              24


<210>  7
<211>  48
<212>  DNA
<213>  artificial

<220>
<223>  CD160 Flag reverse primer

<400>  7
tcacttgtca tcgtcgtcct tgtagtcgcc tgaactgaga gtgccttc                    48

## Description

The present invention relates to the field of immunology and in particular the use of soluble CD160 for the suppression of unwanted immune response. Administration of soluble CD160 is in particular useful for the treatment of an inflammatory condition such as an autoimmune disorder or a tissue or organ rejection. The present invention further relates to a method for screening an individual for the presence of an inflammatory condition such as infectious and autoimmune diseases, tissue graft and organ rejection, or the presence of a tumor, or for monitoring therapy of an inflammatory condition such as an autoimmune disorder or a tissue or organ rejection.

The immune system comprises both the innate immune system and the adaptative or acquired immune system.

The innate immune response is often referred to as a non specific one that controls an invading external noxient until the more specific adaptative immune system can marshal specific antibodies and T cells. The innate immune system includes, for example, natural killer (NK) cells, neutrophils and monocytes/macrophages. NK cells have been implicated in the killing of tumor cells and are essential in the response to viral infections. Another important mechanism of the innate immune system is the activation of cytokine mediators and chemiokines that alert other cells of the presence of infection.

The adaptative immune system comprises antibody-mediated immunity called humoral immunity and regulated by B cells, and cell-mediated immunity controlled by T cells. Both humoral and cell-mediated immunity participate in protecting the host from invading organisms. This interplay can result in effective killing or control of foreign organisms. In particular, CD8+ T cells, when recognizing an antigen bound to a MHC class I molecule, differentiate into cytotoxic T cells, expressing especially granzyme B and perforin, and are therefore able to kill the infected cells.

Occasionally, however, the immune system can become erratic, and this deregulation results in inflammatory conditions, such as autoimmune diseases and organ rejection.

Diverse cytotoxic agents are used in the treatment of an inflammatory condition such as autoimmune diseases and tissue graft or organ rejection, or graft versus host diseases, to depress the host's immune response to a foreign graft or immunogen, or the host's production of antibodies against "self". For example, therapeutic agents having strong suppressive effect against T cells, such as cyclosporine or FK-506, anti-cytokine agents, anti-adhesion molecule agents, or various monoclonal antibodies, are used in these treatments.

The present invention aims to provide an alternative means for the suppression of an undesirable immune response and especially for the treatment of inflammatory conditions including autoimmune diseases and tissue graft or organ rejection.

Accordingly, the Applicant focused on the role of CD160 and especially soluble CD160.

CD160 is a multimeric glycosylphosphatidylinositolanchored lymphocyte surface receptor which expression is mostly restricted to the highly cytotoxic CD56^{dim}CD16⁺ peripheral blood subset in human. CD160 is also expressed in human by most of TCRγδ cells, a subset of TCRαβ CD8^{bright+} T cells and almost all intestinal intraepithelial lymphocytes (iIELs) [Maiza et al. J Exp Med 1993; 178:1121-6; Anumanthan et al. J Immunol. 1998, 161:2780-90]. It has previously been reported that MHC class I molecules bind to CD160 on circulating NK lymphocytes, and that their interaction triggers their cytotoxic activity and cytokine production [Le Bouteiller at al. PNAS 2002; 99(26):16963-8].

WO98/21240 disclosed the nucleic acid and amino acid sequences encoding human BY55, now called CD160, and methods to modulate, particularly to inhibit, expression or activity of CD160 in specific cells. Such methods comprise the administration of a nucleic acid encoding a competitor or an antagonist of CD160 to inhibit CD160 activity. For example, the CD160 domain responsible for its activity can be altered and the altered protein thus obtained can compete with the native CD160 to thereby inhibit its activity. An example of antagonist of CD160 is a nucleic acid capable of inhibiting translation of CD160.

More recently, Tsujimura et al. [Tsujimura et al. Immunology letter 2006, available online] prepared anti-murine CD160 monoclonal antibodies (mAbs) and demonstrated that murine CD160 is expressed on almost all iIELs and a minor subset of CD8+ T cells, as well as NK and NKT cells, as reported previously [Maïza et al. J Exp Med 1993; 178:1121-6; Anumanthan et al. J Immunol. 1998, 161:2780-90; Maeda et al. J Immunol 2005; 175:4426-32]. Tsujimura et al. also found that CD160 is preferentially expressed on memory CD8+ T cells. In addition, they showed that both CD8+ from the spleen and iIELs secrete soluble CD160 upon activation, but this was without any influence on the proliferative response of T cells induced by anti-CD3 mAb. Tsujimura et al. concluded from their study that murine CD160 so far do not seem to have a significant role in the function of CD8+ iIELs and T cells in the periphery, although it is a useful marker for antigen-experienced CD8+ T cells.

The Applicant made the unexpected following observation: the activation of an immune response, mediated both by the innate and the adaptative immune systems, leads to the release of a soluble form of CD160 from cells expressing CD160 such as for example NK cells, T cells, mast cells, or activated endothelial cells. This soluble form of CD160 can then bind to classical and non classical MHC class I molecules and CD1 molecules, resulting in the inhibition of the cytotoxic CD8+ T cells activity, of the CD160-mediated NK cell activity and of TCRγδ and NKT functions.

Therefore, the present invention relates to a means suppressing unwanted immune responses, said means being the soluble form of CD160. The present invention also relates to the use of said means for treating an inflammatory condition involving an undesirable immune response such as autoimmune diseases and tissue graft or organ rejection. The present invention relates also to the use of said means as a marker for the presence of an inflammatory condition or a tumor. The present invention further relates to method for screening a subject for an inflammatory condition, and method for monitoring therapy for an inflammatory condition. The present invention also relates to kits for carrying out such methods.

The present invention will be better understood with the following definitions.

The term "inflammatory condition" is known in the art and as used herein generally refers to any inflammatory cell mediated disease, including infectious (bacterial and viral) and autoimmune diseases. Infectious diseases generally refer to diseases caused by a virus or a bacterium. Examples of viruses causing an infectious disease include but are not limited to HIV-1 virus, herpes simplex, cytomegalovirus, Epstein-Barr virus, HTLV-1 leukaemia virus. Examples of bacterial infectious diseases include but are not limited to syphilis and tuberculosis. Autoimmune diseases generally refer to diseases in which the immune system is overactive and has lost the ability to distinguish between self and non self. Non-limiting examples of inflammatory diseases include allograft rejection, rheumatoid arthritis, osteoarthritis, infectious arthritis, psoriatic arthritis, polychondritis, periarticular disorders, colitis, pancreatitis, system lupus erythematous, inflammatory bowel diseases, multiple sclerosis, conjunctivitis, diabetes, dermatitis, atopic dermatitis, psoriasis, asthma, systemic sclerosis, septic shock, allergies, anaphylaxis, systemic mastocytosis, and infectious diseases of the internal organs such as hepatitis or ulcers.

The term "inflammatory condition involving an unwanted immune response" generally refers to diseases in which the immune system is overactive and has lost the ability to distinguish between self and non self, such as autoimmune diseases or diseases where an immune response is not desired such as tissue graft and organ rejection, graft versus host diseases.

The term "antibody" is known in the art and as used herein generally refers to all types of immunoglobulins, including IgG, IgM, IgA, IgD, and IgE. The term "immunoglobulin" includes the subtypes of these immunoglobulins, such as IgG1, IgG2, IgG3... The antibodies may be of any species of origin, including for example mouse, rat, rabbit, horse, or human, or may be chimeric or humanized antibodies. Monoclonal antibodies are produced in accordance with known techniques. The term "antibody" or "antibodies" as used herein includes antibody fragments which retain the capability of binding to a target antigen, for example, Fab, F(ab')2, and Fv fragments, and the corresponding fragments obtained from antibodies other than IgG. Such fragments are also produced by known techniques.

The term "immunosuppressive agent" is known in the art and as used herein generally refers to a medication that slows or halts immune system activity. Immunosuppressive agents may be given to prevent the body from mounting an immune response after an organ transplant or for treating a disease that is caused by an overactive immune system. Immunosuppressive agents include but are not limited to substances that suppress cytokine production, downregulate or suppress self-antigen expression, or mask the MHC antigens. Examples of such agents are glucocorticoids, cytostatics, antibodies, drugs acting on immunophilins and interferons, opoids, TNF binding proteins. Such immunosuppressive agents include but are not limited to 2-amino-6-aryl-5-substituted pyrimidines, azathioprine (or cyclophosphamide), bromocryptine, glutaraldehyde, anti-idiotypic antibodies for MHC antigens and MHC fragments, cyclosporine A, steroids such as glucocorticosteroids (prednisone, methylprednisone, dexamethasone), cytokine and cytokine receptor antagonists including interferon-gamma, - beta, or -alpha antibodies, anti-tumor necrosis factor antibodies, anti-interleukine-2 antibodies and anti-IL-2 receptor antibodies, anti-L3T4 antibodies, heterologous antilymphocyte globulin, pan-T antibodies, preferably anti-CD3 or anti-CD4 antibodies, soluble peptide containing a LFA-3 binding domain, streptokinase, TGF-beta, streptodomase, FK506, RS-61443, deoxyspergualin, rapamycin, T cell receptor, T cell receptor fragments and T cell receptor antibodies such as T10B9. Preferably the immunosuppressive agent comprises cyclosporine A, FK506, rapamycin, steroids such as glucocorticosteroid (most preferably prednisone or methylprednisolone), cytostatics such as methotrexate, azathioprine, and monoclonal antibodies such as OKT3 or anti-TNF.

The term "graft" is known in the art and as used herein generally refers to biological material derived from a donor for transplantation into a recipient or host. Grafts include such diverse material as, for example, isolated cells such as islet cells and neural-derived cells, tissue such as the amniotic membrane of a newborn, bone marrow, hematopoietic precursor cells, and organs such as skin, heart, liver, spleen, pancreas, thyroid lobe, lung, kidney, tubular organs... The graft is derived from any mammalian source, including human. In some embodiments, the graft is preferably bone marrow or an organ such as heart, kidney or liver.

The term "transplant" or "transplantation" is known in the art and as used herein generally refers to the insertion of a graft into a host, whether the transplantation is syngeneic (where the donor and recipient are genetically identical), allogeneic (where the donor and recipient are of different genetic origins but of the same species), or xenogeneic (where the donor and recipient are from different species). Typically, the host is human and the graft is an isograft, derived from a human of the same or different genetic origins.

By "individual", it is meant mammal, in particular a human being.

As used herein, "treatment" or "treating" generally refers to a clinical intervention in an attempt to alter the natural course of the individual or cell being treated, and may be performed either for prophylaxis or during the course of clinical pathology. Desirable effects include, but are not limited to, preventing occurrence or recurrence of disease, alleviating symptoms, suppressing, diminishing or inhibiting any direct or indirect pathological consequences of the disease, preventing metastasis, lowering the rate of disease progression, ameliorating or palliating the disease state, and causing remission or improved prognosis.

It is an object of the present invention to provide a pharmaceutical composition comprising a soluble form of CD160.

"soluble form" as used herein include truncated form, which is one where the molecule has been cleaved at the GPI linkage, and any other form which has been deleted of amino acids residues that bind the protein to or into the cell membrane.

"soluble form of CD160" as used herein refers to the extracellular part of CD160 (amino acids 1 to 160, Genbank accession number AF060981 (human) or AF060982 (mouse)), and fragments and derivatives thereof.

The term "fragment" when referring to soluble CD160 means proteins which retain essentially the same biological function or activity as the protein CD160. This biological function or activity of the CD160 comprises the binding to a classical or non-classical MHC class I molecule or to a CD1 molecule. Therefore, for that function, fragments and derivatives of a soluble form of CD160 of the present invention maintain at least about 50% of the activity of the protein CD160, preferably at least 75% and more preferably at least 95%.

A soluble CD160 fragment or derivative may be (i) a peptide in which one or more of the amino acids residues are substituted with a conservative or non-conservative amino acid residue (preferably a conservative amino acid residue) and such substituted amino acid residue may or may not be one encoded by the genetic code, or (ii) a peptide in which one or more of the amino acids residues includes a substitute group, or (iii) a peptide in which the mature protein is fused with another compound , such as a compound to increase the half-life of the polypeptide (for example, polyethylene glycol). For example, a soluble form of CD160 may be used to form a fusion protein with an immunoglobulin.

In one embodiment of the invention, said pharmaceutical composition comprises a soluble form of CD160 in combination with at least one acceptable carrier and optionally other therapeutic ingredients.

The carrier(s) must be "acceptable" in the sense of being compatible with the others ingredients of the formulation and not deleterious to the recipient thereof.

According to the invention, said pharmaceutical composition includes those suitable for oral, rectal, nasal, topical and parenteral (including subcutaneous, intramuscular, intravenous, and intradermal) administration. Preferably, parenteral administration of said pharmaceutical composition is used. The formulations may conveniently be presented in unit dosage form such as tablets and sustained release capsules, and in liposomes or immunopastides, and may be prepared by any method well known in the art.

Pharmaceutical composition according to the invention and suitable for oral administration may be presented as discrete units such as capsules, microcapsules, cachets or tablets each containing a predetermined amount of the active ingredient, as a powder or granules, as a solution or a suspension in an aqueous liquid or non-aqueous liquid, or in colloidal drug delivery system (for example, liposomes, albumin microspheres, microemulsion, nano-particles and nanocapsules) or in macroemulsion. Such techniques are disclosed in Remington's Pharmaceutical Sciences 16th edition, Osol, A. Ed. (1980).

Pharmaceutical composition according to the invention and suitable for parenteral administration include aqueous or non-aqueous sterile injection solutions which may comprise anti-oxidants, buffer, bacteriostats and solutes which render the formulation isotonic with the blood of the intended recipient; and aqueous or non-aqueous sterile suspensions which may include suspending agents and thickening agents. The formulations may be presented in unit-dose or multi-dose containers, for example sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Pharmaceutical composition according to the invention may also be administrated locally at the site of interest. Various techniques can be used for providing the pharmaceutical composition at the site of interest, such as injection, use of catheters, trocars, projectiles, pluronic gel, stents, sustained drug release polymers or other device which provides for internal access. Where an organ or tissue is accessible because of the removal from an individual, such organ or tissue may be bathed in a medium containing said pharmaceutical composition, the pharmaceutical composition may be painted onto the organ, or may be applied in any convenient way. Systemic administration using for example liposomes with tissue targeting such as an antibody may also be employed.

It is also an object of the present invention to provide said pharmaceutical composition comprising a soluble form of CD160 for treating an inflammatory condition involving an unwanted immune response, such as tissue graft or organ rejection, and autoimmune diseases.

Without wanting to be bound to any theory, the administration to an individual in need thereof of a pharmaceutical composition comprising a soluble form of CD160 should prevent the classical and non classical MHC class I molecules, and CD1 molecules to be recognized by cytotoxic CD8+ T cells, NK cells, NKT and TCRγδ cells, thereby resulting in the inhibition of the cytotoxic CD8+ T cells activity, of the CD160-mediated NK cell activity and of NKT and TCRγδ functions. This effect should result in the inhibition of the unwanted immune response.

In a preferred embodiment of the invention, said pharmaceutical composition permits the treatment of organ rejection such as heart, kidney or liver rejection.

In another preferred embodiment of the invention, said pharmaceutical composition permits the treatment of inflammatory diseases such as rheumatoid arthritis, atopic dermatitis, psoriasis, multiple sclerosis, diabetes, lupus and inflammatory bowel diseases.

The present invention further relates to the use of a soluble form of CD160 for the preparation of a pharmaceutical composition for treating inflammatory conditions, including tissue graft or organ rejection, and autoimmune diseases.

In a preferred embodiment, the invention relates to the use of a soluble form of CD160 for the preparation of a pharmaceutical composition for treating organ rejection such as heart, kidney or liver rejection.

In a preferred embodiment, the invention relates to the use of a soluble form of CD160 for the preparation of a pharmaceutical composition for treating inflammatory diseases such as rheumatoid arthritis, atopic dermatitis, psoriasis, multiple sclerosis, diabetes, lupus and inflammatory bowel diseases.

Certain embodiments of this invention relate to combination therapies. In one embodiment, the pharmaceutical composition of the invention is used in combination with at least one immunosuppressive agent for treating inflammatory conditions. Preferably, said at least immunosuppresive agent comprises cyclosporine A, FK506, rapamycin, steroids such as glucocorticosteroid (most preferably prednisone or methylprednisolone), cytostatics such as methotrexate, azathioprine, and monoclonal antibodies such as OKT3 or anti-TNF.

Immunosuppressive agents are used in immunosuppressive therapy to inhibit or prevent activity of the immune system. They are capable of suppressing the cell mediated immunity and the humoral immunity and inhibiting various inflammatory events. The use of the pharmaceutical composition according to the invention in combination with said immunosuppressive agent should permit to suppress specifically the T and NK cells mediated response and therefore to enhance the beneficial effect of the immunosuppressive therapy.

It is another object of the present invention to provide a kit for treating an inflammatory condition comprising a pharmaceutical composition as described above and at least one immunosuppressive agent as described above.

In a preferred embodiment, the invention provides said kit for treating tissue graft or organ rejection.

The present invention relates to an in vitro method for screening the presence of an inflammatory condition such as infectious and autoimmune diseases, tissue graft and organ rejection, or the presence of a tumor or activated endothelial cells, wherein a soluble CD160 is used as a marker

Indeed, the applicant observed that the activation of an immune response, mediated both by the innate and the adaptative immune systems, leads to the release of a soluble form of CD160 from cells expressing CD160 such as for example NK cells, T cells, mast cells, activated endothelial cells. Therefore, the presence of high level of soluble CD160 in a biological sample from an individual should indicate the presence of an immune response, which can be observed in infectious and autoimmune diseases, in tissue graft or organ rejection and in the presence of a tumor or activated endothelial cells.

The present invention relates to an in vitro method for monitoring therapy of an inflammatory condition such as an autoimmune disorder or a tissue or organ rejection or for monitoring the presence of a tumor during chemotherapy including treatment with an anti-angiogenic substance or antibody, wherein a soluble CD160 is used as a marker.

The treatment of an inflammatory condition such as an autoimmune disease or a tissue or organ rejection can thus be monitored by the level of soluble CD160. The presence of high level of soluble CD160 in a biological sample from an individual treated with an immunosuppressive agent should indicate that the unwanted immune response is not suppressed. In the same way, the treament of a tumor with chemotherapy including treatment with an anti-angiogenic substance or antibody can be monitored y the level of soluble CD160. The presence of high level of soluble CD160 in a biological sample from an individual treated against a tumor should indicate that the tumor is still present and capable to induce an immune response.

In a preferred embodiment, said methods described above are used for screening or for monitoring therapy of an organ rejection such as heart, kidney or liver rejection.

In a preferred embodiment, said methods described above are used for screening or for monitoring therapy of inflammatory diseases such as rheumatoid arthritis, atopic dermatitis, psoriasis, multiple sclerosis, diabetes, lupus and inflammatory bowel diseases.

Level of soluble CD160 can be detected in a biological sample from an individual and compared to the level of soluble CD160 from a healthy control population. The level of soluble CD160 from a control population generally refers to the average level of soluble CD160 from a plurality of individuals without an inflammatory condition, a tissue graft or organ rejection or a tumor.

Suitable biological samples for measuring soluble CD160 levels include for example blood (including whole blood, plasma and serum), urine, cerobrospinal fluid, joint effusion, ascites, amniotic fluid. Serum is preferably used as a biological sample.

The presence of an inflammatory condition such as infectious and autoimmune diseases, of tissue graft or organ rejection, or of a tumor or activated endothelial cells can be determined based on the level of soluble CD160 relative to the control population. Thus, it is determined if the level of soluble CD160 is increased, similar or decreased compared to the one observed in the control population. An increase in soluble CD160 level relative to that of the control population is indicative of an inflammatory condition such as infectious and autoimmune condition, of a tissue graft or organ rejection or of the presence of a tumor or activated endothelial cells. Additional factors that can be considered when diagnosing such disorders include for example patient history, family history, genetic factors.

The level of soluble CD160 in an individual also can be used to monitor treatment, such as treatment for said inflammatory conditions or chemotherapy including treatment with an anti-angiogenic substance or antibody. Typically, the individual baseline's level of soluble CD160 is obtained before treatments and compared to the level of soluble CD160 at various time points after or between treatments, for example one or more days, weeks, or months after treatment). A decrease in soluble CD160 level relative to the baseline level is indicative of a positive response to treatment.

Soluble CD160 can be detected for example by immunological assays using one or more antibodies. In these assays, an antibody having a specific binding affinity for soluble CD160 or a secondary antibody that binds to such an antibody can be labelled, either directly or indirectly. Suitable labels include, without limitation, radionuclides (such as ¹²⁵I, ¹³¹I_{'} ³⁵S, ³H, ³P, ³³P or ¹⁴C), fluorescent moieties (such as fluorescein, FITC, PerCP, rhodamine, Alexa, or PE), luminescent moieties (such as Qdot^{™} nanoparticles supplied by the Quantum Dot Corporation, Palo Alto), compounds that adsorb light of a defined wavelength, or enzymes (such as alkaline phosphatise or horseradish peroxidise). Antibodies can be indirectly labelled by conjugation with biotin then detected with avidin or streptavidin labelled with a molecule described above. Methods for detecting or quantifying a label depend on the nature of the label and are known in the art. Examples of detectors include, but are not limited to, x-ray film, radioactivity counters, scintillation counters, spectrophotometers, colorimeters, fluorometers, luminometers, and densitometers. Combinations of these approaches (including "multilayer" assays) familiar to those in the art can be used to enhance the sensitivity of assays.

Immunological assays for detecting soluble CD160 can be performed in a variety of known formats, including sandwich assays, competitions assays or bridge immunoassays.

In one embodiment, said in vitro methods for screening the presence of an inflammatory condition such as infectious and autoimmune diseases, tissue graft and organ rejection, or the presence of a tumor or activated endothelial cells, or for monitoring therapy of an inflammatory condition such as an autoimmune disorder or a tissue or organ rejection comprise:
- contacting a biological sample with a ligand that binds to soluble CD160, and
- detecting the binding of soluble CD160 to said ligand.

In a preferred embodiment, said ligand comprises an antibody that binds to soluble CD160 or one of CD160 receptors such as classical or non classical MHC class I molecules or CD1 molecules.

In one embodiment, said ligand having a specific binding affinity for soluble CD160 can be immobilized on a solid substrate by any variety of methods known in the art and exposed to the biological sample. The binding of soluble CD160 to the ligand on the solid substrate can be detected by exploiting the phenomenon of surface plasmon resonance, which results in a change in the intensity of surface plasmon resonance upon binding that can be detected qualitatively or quantitatively by an appropriate instrument, such as a Bioacore apparatus (BIAcore International AB, Rapsgatan, Sweden). Alternatively, the ligand can be labelled and detected as described above. A standard curve using known quantities of soluble CD160 can be generated to aid in the quantification of soluble CD160 level.

In another embodiment, a "sandwich" assay in which a capture antibody is immobilized on a solid substrate is used to detect the level of soluble CD160. The capture antibody includes, but is not limited to, an antibody that binds to soluble CD160 or a recombinant antibody comprising a Fc fragment or immunoglobulin constant region and a soluble human classical or non classical MHC class I or human CD1.

The solid substrate can be contacted with the biological sample such that any soluble CD160 in the sample can bind to the immobilized antibody. The level of soluble CD160 bound to the antibody can be determined using a "detection" antibody having a specific binding affinity for soluble CD160 and the methods described above. It is understood that in these sandwich assays, the capture antibody should not bind to the same epitope (or range of epitopes in the case of a polyclonal antibody) as the detection antibody. Sandwich assays can be performed as sandwich ELISA assays, sandwich Western blotting assays, or sandwich immunomagnetic detection assays.

Suitable solid substrates to which an antibody such as a capture antibody, can be bound include, but are not limited to, microtiter plates, tubes, membranes such as nylon or nitrocellulose membranes, and beads or particles such as agarose, cellulose, glass, polystyrene, polyacrylamide, magnetic, or magnetisable beads or particles). Magnetic or magnetisable particles can be particularly useful when an automated immunoassay system is used.

Alternative techniques for detecting soluble CD160 include mass-spectrophotometric techniques such as electrospray ionization (ESI), matrix-assisted laser desorption-ionization (MALDI). Mass spectrophotometers useful for such applications are available from Applied Biosystems, Bruker Daltronics and Amersham Pharmacia.

In one embodiment of the present invention, the level of soluble CD160 is detected using a monoclonal antibody.

In one embodiment of the present invention, the level of soluble CD160 is detected using a capture antibody and a detection antibody, wherein said detection antibody comprises a label. Said capture antibody is preferably attached to a solid substrate, said solid substrate comprises a bead or a microtiter plate.

The present invention also relates to a kit for detecting soluble CD160 from a biological sample, for screening the presence of an inflammatory condition such as infectious and autoimmune diseases, tissue graft and organ rejection, or the presence of a tumor or activatd endothelial cells, or for monitoring therapy of an inflammatory condition such as an autoimmune disorder or a tissue or organ rejection, or for monitoring the presence of a tumor during chemotherapy including treatment with an anti-angiogenic substance or antibody,said kit comprising:
- at least one ligand having a specific binding affinity for soluble CD160, said ligand comprising an antibody or a classical or non classical MHC class I molecule or a CD1 molecule, and
- reagents such as secondary antibodies.

The invention will be further described in the following examples, which do not limit the scope of the invention described in the claims.

### DESCRIPTION OF THE DRAWINGS

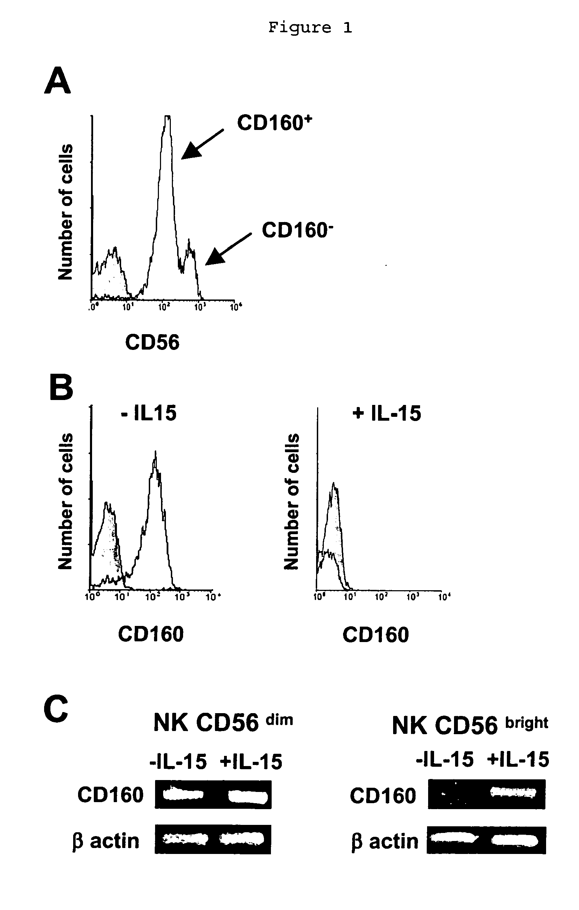

**Figure 1. Down-expression of CD160 at the cell surface of IL-15-activated PB-NK lymphocytes.** (A) CD56 expression level delineates two subpopulations of NK cells. PB-NK cells were isolated from the PBMC of a healthy donor and immunolabeled using a PE-conjugated control IgG or anti-CD56 mAb. Flow cytometry cell analysis was conducted and allowed the detection of the CD56^{dim} CD160⁺ (≥ 90%) and CD56^{bright} CD160⁻ (≤ 10%) subpopulations, as indicated. (B) Flow cytometry analysis of membrane-associated CD160 on resting and IL-15-treated PB-NK lymphocytes. PB-NK cells were cultured in medium alone (left panel) or supplemented with IL-15 (10ng/ml; right panel) for 72 h. Cells were labeled with either the BY55 and anti-IgM FITC-coupled secondary antibodies or the CL1-R2 and anti-IgG FITC-coupled secondary antibodies, and further analyzed by flow cytometry. (C) Analysis of CD160 mRNA synthesis in PB-NK cell subpopulations. CD56^{dim} and CD56^{bright} NK cell subsets were obtained from purified PB-NK lymphocytes by immunostaining with an anti-CD56 PE-conjugated mAb followed by a cell sorting procedure. Each subpopulation was then maintained in culture in the absence (- IL-15) or presence (+ IL-15) of cytokine for 72 h. Total mRNA was extracted from each cell type and processed for reverse-transcription and CD160 cDNA specific amplification. Amplification of the same reverse-transcribed product with β-actin primers was used as internal control.

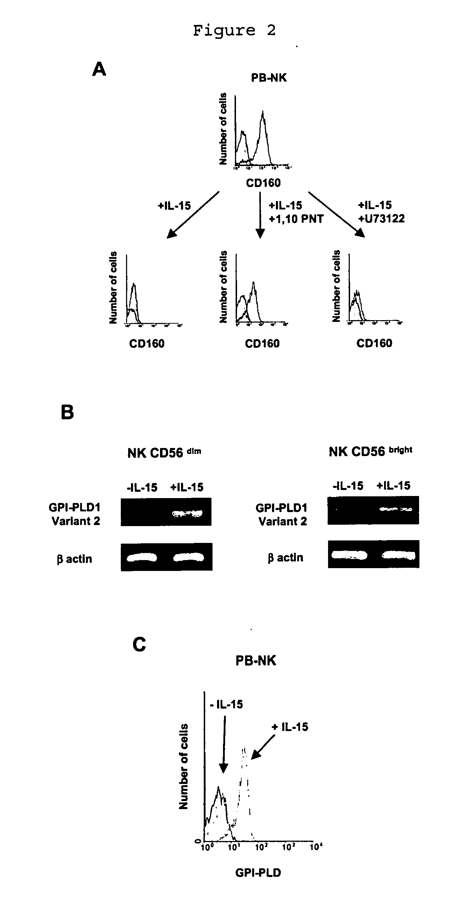

**Figure 2. The down-modulation of CD160 cell surface expression on IL-15-activated PB-NK lymphocytes involves a Zn²⁺-dependent protease and correlates with the neo-synthesis of the GPI-PLD enzyme.** (A) Inhibition of the IL-15-induced CD160 down-expression in the presence of 1,10 PNT. Freshly isolated PB-NK cells were cultured in IL-15-containing medium alone, or supplemented with the phospholipase inhibitor 1,10 PNT (10 µM) or U73122 (2 µM). Membrane-bound CD160 was detected by flow cytometry analysis with the anti-CD160 mAb BY55 plus anti-IgM-FITC antibodies. A control IgM was used as negative control (B) Expression of CD160 mRNA following IL-15-activation of NK cells. Sorted CD56^{dim} or CD56^{bright} NK cells were grown in culture medium ± IL-15 for 72 h prior to total mRNA extraction. After reverse-transcription, cDNA amplification was performed using primer pairs leading to the synthesis of products corresponding to the specific coding sequence of the GPI-PLD1 variant 2 and β-actin. (C) The IL-15 treatment induces the synthesis of GPI-PLD by PB-NK cells. Resting or IL-15-activated PB-NK lymphocytes were subjected to a permeabilization step in saponin-containing buffer before immunolabeling. Cells were then incubated with specific anti-GPI-PLD or control antibodies, and FITC-coupled secondary reagent. The intracellular protein immunostaining was further detected by flow cytometry analysis.

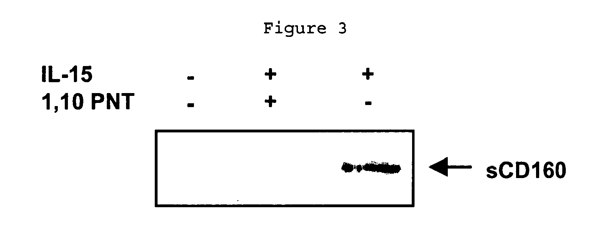

**Figure 3. Detection of soluble CD160 molecules within the extracellular environment of IL-15-stimulated PB-NK cells.** PB-NK cells were left untreated or activated for 72 h with IL-15, then washed and incubated with IL-15 alone or supplemented with 1,10 PNT protease inhibitor. Cell culture supernatants were collected and subjected to anti-CD160 immunoprecipitation using CL1-R2 mAb. Following protein separation by SDS-8% PAGE under reducing conditions, the precipitated proteins were transferred onto a nitrocellulose membrane and subjected to anti-CD160 immnoblotting. The immunoreactive proteins were visualized using HRP-conjugated secondary antibodies and an ECL detection system.

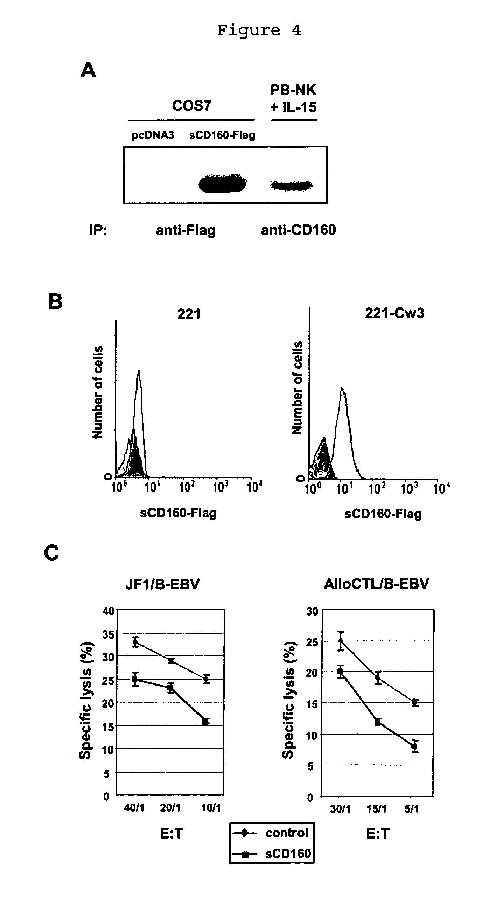

**Figure 4. sCD160 inhibits the cytolytic activity of NK and allogeneic CD8⁺ cytotoxic T lymphocytes towards an EBV-B cell line.** (A) The *in vitro* produced sCD160-Flag protein structurally corresponds to sCD160 molecules released by activated PB-NK cells. COS7 cells were transiently transfected with the pcDNA3 control vector or with the expression construct encoding a Flag-tagged soluble CD160 protein (sCD160-Flag). Following a 2-days culture, the cell supernatants were collected and anti-Flag immunoprecipitates were prepared. An anti-CD160 immunoprecipitation was performed in parallel on the culture medium from IL-15-treated PB-NK lymphocytes. Immunoprecipitates were resolved by reducing SDS-8% PAGE, and subjected to Western blot analysis using the anti-CD160 mAb CL1-R2. Proteins were finally detected by autoradiography as described in the legend of Fig. 3. (B) sCD160-Flag fusion protein efficiently binds to HLA-C molecules. The parental cell line 221 or the HLA-Cw3 expressing transfectants (221-Cw3) were incubated with culture medium obtained from COS7 cells transfected with pcDNA3 (negative control) or sCD160-Flag coding vector. A 50g/ml concentration of sCD160-Flag-containing medium was used in the shown experiment. The binding of sCD160-Flag to the cells was assessed by immunostaining using the anti-Flag mAb and anti-IgG FITC-coupled antibodies, and subsequent flow cytometry analysis. (C) sCD160 inhibits the cytotoxic activity of CTLs. Cytotoxic assays were conducted using ⁵¹Cr-loaded HLA-A11-expressing EBV-transformed B cells as target cells. The target cells were incubated with control or sCD160-Flag-containing culture medium from transfected COS7 cells prior to contact with the effector cells. The HLA-A11-specific human cytotoxic T cell clone JF1 (left panel), or sorted allogeneic CD8⁺ cytotoxic T lymphocytes (right panel), were selected as effector cells. Each experimental condition was performed in triplicate and included three different effector/target cell ratios. Cr⁵¹-release was measured in the co-culture supernatants, and results were expressed as the % of specific lysis ± SD.

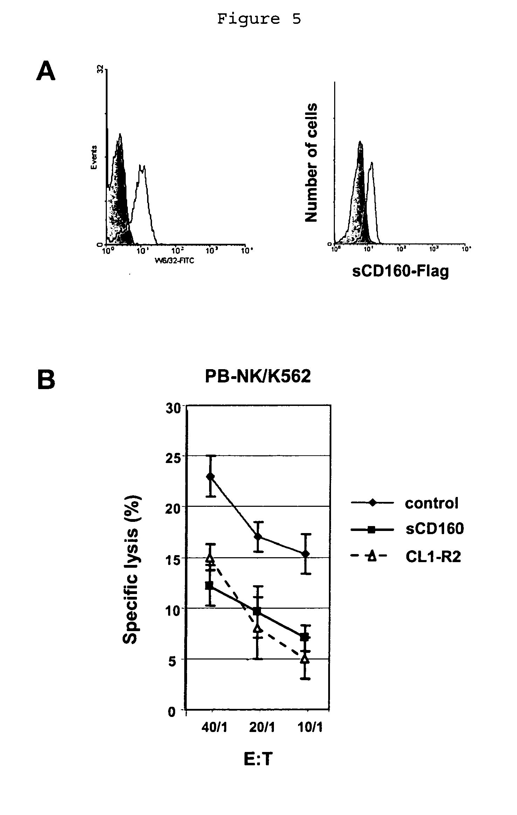

**Figure 5. The interaction of sCD160 with MHC-I molecules on K562 cells down-regulates the PB-NK cell cytotoxic activity.** (A) Binding of sCD160-Flag to K562 cells. K562 cells were either labeled with the anti-MHC I molecules mAb W6/32 (left panel) or subjected to sCD160-Flag binding assay as described in the legend of Fig. 4B. (B) K562 cells were pre-incubated with control or sCD160-Flag-containing medium before contact with the effector PB-NK lymphocytes. Alternatively, the anti-CD160 mAb CL1-R2 was added to the effector cells before starting the co-culture. K562 cell lysis was quantified as explained in the Material and Methods section.

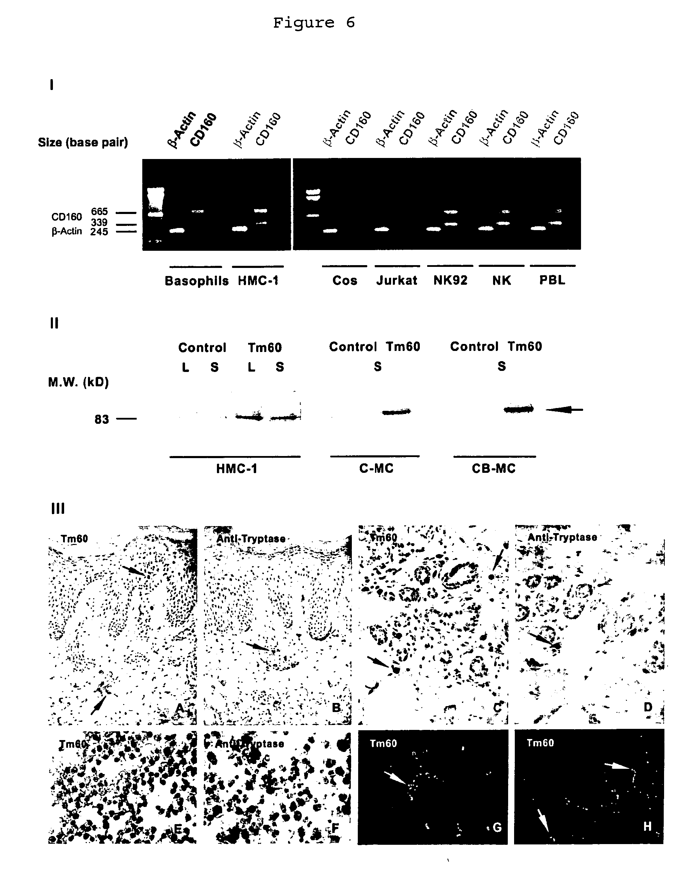

**Figure 6. The expression of CD160 and production of soluble CD160 by mast cells.**

RT-PCR was done using standard procedure and specific primers for β actin and CD160. mRNA from cell lines and peripheral leukocytes were purified using the Trizol ragent technique. HMC-1 cells as well as peripheral basophils from a healthy donor display CD160 mRNA expression, with the two alternatively spliced short and long transcripts of 339 and 665 base pair, respectively. A similar expression is found in PBL and NK cells from healthy donors and in NK92 cells. As negative controls, Cos and Jurkat cells do not show CD160 expression.

CD160 immunoprecipitation was done in HMC-1 cells lysate and supernatant, as well as in supernatant of human mast cells grown in culture, from CD34⁺ pluripotent progenitor derived from cord blood (CB-MC) or cytapheresis (C-MC) of healthy donors. Following protein separation, immunoblotting was performed using the Tm60 monoclonal antibody and a horseradish peroxidase-conjugated secondary antibody. A specific 83 kD immunoprecipitate is found in the supernatant of both HMC-1 cells and CD34+ progenitor cells derived human mast cells, indicating that these cells produce soluble CD160.

Standard immunohistochemical detection of CD160 and mast cell tryptase was done with the avidin biotin-peroxydase technique, using Tm60 monoclonal antibody (CL1-R2) (A, C, E) and (B, D, F). For immunofluorescent tests, secondary biotinylated anti-mouse IgG antibodies and fluoroisothiocyanate conjugated streptavidine were used (G, H). In normal skin and accessory salivary gland paraffin embedded tissue sections, mast cells are strongly stained with anti-CD160 monoclonal antibodies (A, C, arrows). In the skin, they are located in the papillary dermis and around dermal capillaries of the mid and deep dermis (A). In salivary gland, they are seen around the salivary ducts and acini (C). In both skin and salivary gland, mast cells are characterized morphologically and by mast cell tryptase expression (B, D). HMC-1 cells strongly express CD160, especially within cytoplasmic granules (E), as well as mast cell tryptase (F). Using immunofluorescent tests, mast cells from cutaneous mastocytosis (G) and HMC-1 cells (H) display strong granular expression of CD160 (arrows).
(Magnifications : A,B,C,D,E,F X200 ; G, H : X400)

### EXAMPLES

### Materials and Methods

### Cells

PBMC were isolated from heparinized venous blood obtained from healthy donors, by density gradient centrifugation over MSL (PAA Laboratories, Les Mureaux, France). Fresh PB-NK cells were isolated using a magnetic-activated cell sorter (MACS) and a NK cell isolation kit according to the manufacturers' recommendations (Miltenyi Biotec, Bergish-Gladbach, Germany). PB-NK cell purity was shown to be >90%. The selection of in vitro allogeneic-MHC class I-restricted effector T lymphocytes was performed as previously reported (Bensussan, A., B. Tourvieille, L-K. Chen, J. Dausset, and M. Sasportes. 1985. Proc. Natl. Acad. Sci. USA 82: 6642-6646.). Briefly, PBMC were co-cultured for 6 days with irradiated EBV-transformed B cells in RPMI 1640 medium supplemented with penicillin (100 IU/ml), streptomycin (100 µg/ml), L-glutamine (2 mM), and 10% heat-inactivated human serum (Jacques Boy Institute, Lyon, France). At day 6 of the mixed lymphocyte culture, the CD8⁺ population was isolated using CD8⁺ microbeads according to the manufacturer's instructions (Miltenyi Biotec). Cells were then extensively washed and cultured overnight at 37°C, and further tested as effector cells against the specific allogeneic EBV-B target cell line in lymphocyte-mediated cytotoxicity assays. CD8⁺ cells represented more than 90% of the isolated lymphocyte population. To separate the CD56^{dim} and CD56^{bright} PB-NK cell populations, PB-NK. cells were stained with an anti-CD56 PE-conjugated mAb and sorted using an ELITE cell sorter (Beckman-Coulter, Miami, FL).

All cell lines used in this study were cultured in standard culture medium containing 10% fetal calf serum (FCS; Perbio Science, Brebières, France). The 721.221-HLACw3 stable transfectants (221-Cw3, kindly provided by Dr Philippe Le Bouteiller, INSERM U563, Toulouse, France) were obtained by transfection of 721.221 cells (221) with a HLA-Cw3 coding vector.

### Antibodies and flow cytometry

The antibodies used in this study were the following: anti-Flag M2 mAb (Sigma, St Quentin Fallavier, France), rabbit anti-GPI-phospholipase D (Caltag laboratories, Burlingame, CA), anti-CD56 mAb (Beckman-Coulter, Marseille, France), and anti-CD160 mAb (BY55 (IgM) and CL1-R2 (IgG1), produced locally). Irrelevant isotype-matched antibodies were used as negative controls. FITC- or PE-conjugated goat anti-mouse IgG or IgM (Beckman-Coulter), or goat anti-rabbit IgG (Caltag laboratories) were utilized as secondary reagents. Cells were phenotyped by indirect immunofluorescence. Briefly, the cells were incubated with the specific mAb for 30 min at 4°C, washed twice in PBS, and further incubated with the appropriated FITC- or PE-labeled secondary antibodies. After washing, cells were analyzed by flow cytometry on an EPICS XL apparatus (Beckman-Coulter). For intracellular staining, the cells were permeabilized in saponin buffer (PBS/0.1% BSA/0.1% saponin) (Sigma) prior to staining, and all subsequent steps were performed in saponin buffer, as described above.

For soluble CD160 (sCD160) binding assays, COS7 cells were transfected with an expression vector coding for a Flag-tagged soluble CD160 protein. Following cell recovery, several dilutions of COS7 cell culture medium, corresponding to concentrations of sCD160 ranging from 0.5 to 10 µg/ml were tested for their ability to label HLA-Cw3 expressing cells. Typically, 10⁵ 221-Cw3 cells were incubated with 50 µl of sCD160-Flag containing supernatant in a 96 well round-bottomed plate. Culture medium obtained from COS7 cells transfected with an empty vector was used as negative control. After 1h at 37°C, cells were washed and fixed in PBS containing 2% paraformaldehyde for 20 min at 4°C. Cells were then washed twice, incubated with the anti-Flag M2 mAb for 20 min at 4°C, and stained with FITC-conjugated goat anti-mouse antibodies. After washes, the cells were analyzed using an XL flow cytometer (Beckman-Coulter).

### Inhibition of the GPI-anchored CD160 cleavage

The phospholipase inhibitors U73122, U73343 and 1,10 phenanthroline (1,10 PNT) were purchased from Sigma. 3 x 10⁵/ml PB-NK cells were cultured for 72 h in RPMI 1640 supplemented with 10% heat-inactivated human serum, penicillin/streptomycin, L- glutamine and IL-15 (10 ng/ml; PeproTech, Levallois-Perret, France). The cells were then washed and incubated for 8-12 h at 37°C in IL-15 containing medium alone, or supplemented with 1,10 PNT (10µM), U73122 (2µM) or U73343 (2µM). U73343, an inactive analog of U73122, was used as negative control. After two washes in PBS, cells were processed for CD160 immunostaining and flow cytometry analysis.

### RNA extraction, reverse transcription and cDNA amplification (RT-PCR)

Total RNA was isolated using the Trizol reagent according to the manufacturer's instructions (Invitrogen, Cergy Pontoise, France). For each reverse transcription, 5*µ*g of RNA were used. Reverse transcription was performed using 500ng of an oligo-dT primer (Invitrogen) and the Powerscript reverse transcriptase (Clontech, Palo Alto, CA) in a total volume of 20*µ*l. Specific primers for the amplification of CD160 and GPI-PLD1 variant 1 and 2 cDNA were designed on the basis of published sequences (Anumanthan, A., A. Bensussan, L. Boumsell, A. D. Christ, R. S. Blumberg, S. D. Voss, A. T. Patel, M. J. Robertson, L. M. Nadler, and G. J. Freeman. 1998. J. Immunol. 161: 2780-2790 ; Schofield, J. N., and T. W. Rademacher. 2000. Biochim. Biophys. Acta. 1494: 189-194.). The CD160 primers were as follows: 5'-TGCAGGATGCTGTTGGAACCC-3' (forward, SEQ ID n°1) and 5'-CCTGTGCCCTGTTGCATTCTTG-3' (reverse, SEQ ID n°2). The primer sequences for the cDNA amplification of GPI-PLD1 variant 1 were 5' ATGGATGGCGTGCCTGACCTGGCC-3 (forward, SEQ ID n°3) and 5'-CAGCGGTGGCTGCAGGTCGGATGT-3' (reverse, SEQ ID n°4), and 5'-GTGTTGGACTTTAACGTGGACGGC-3' (forward, SEQ ID n°5) and 5'-CAGCAGAGGCTGCGCGTCAGATAT-3' (reverse, SEQ ID n°6) for the GPI-PLD1 variant 2. β actin cDNA amplification was performed in parallel as internal control. The synthesis of specific cDNA fragments was achieved by using 1µl of the reverse-transcribed product according to a standard procedure (Invitrogen), in a total volume of 20µl. Each sample was subjected to denaturation (94°C, 30 sec), annealing (60°C, 30 sec), and extension (72°C, 90 sec) steps for 35 cycles. The amplified products were separated on a 1% agarose gel.

### Production and quantification of soluble CD160-Flag

A cDNA encoding a C-terminal Flag (DYKDDDK)-tagged soluble CD160 (sCD160-Flag) was generated by PCR amplification of the sequence corresponding to amino-acids 1-160 of CD160 with the following primers: 5'- TGCAGGATGCTGTTGGAACCC-3' (forward, SEQ ID n°1) and 5'-TCACTTGTCATCGTCGTCCTTGTAGTCGCCTGAACTGAGAGTGCCTTC-3' (Flag-reverse, SEQ ID n°7). After purification, the resulting PCR product was ligated into the pcDNA3 expression vector (Invitrogen), and the construct double-strand sequenced.

COS7 cells were transiently transfected with the pcDNA3 vector, or sCD160-Flag expression vector, using the DEAE-dextran method, and subsequently cultured for 72 h in serum free RPMI 1640 medium supplemented with L-glutamine and antibiotics. An ELISA was developed to detect the produced sCD160-Flag protein in the cell culture medium, as previously performed for the quantification of soluble CD100 (Delaire, S., C. Billard, R. Tordjman, A. Chedotal, A. Elhabazi, A. Bensussan, and L. Boumsell. 2001. J. Immunol. 166: 4348-4354). Briefly, the anti-Flag M2 mAb (5 µg/well) was coated in a 96-well plate (MaxiSorp, Nunc, CliniSciences, Montrouge, France) overnight at 4°C. All subsequent steps were performed at 4°C. Following saturation with PBS/1% BSA for 4 h, the sCD160-Flag containing medium of transfected COS7 cells was added for 2 h. After extensive washes with PBS/1% BSA, the anti-CD160 (CL1-R2)-biotinylated mAb (diluted in PBS/1% BSA) was added. After washes and incubation with streptavidin-alkaline phosphatase, the revelation step was performed using the pNpp liquide substrate system for ELISA (Sigma). After 1h incubation in the dark, at room temperature, the absorbance was measured at 405 nm using a plate reader spectrophotometer (Packard, Downers Grove, IL). A standard curve was realized using purified sCD160-Flag protein. To this aim, sCD160-Flag was immunoprecipitated from transfected COS7 culture medium using CL1-R2 mAb coupled to protein G-Sepharose beads (Amersham Biosciences, Orsay, France) and eluted in 2mM glycine-HCl pH 2.8. After neutralization, a second immunoprecipitation step was performed with agarose-coupled anti-Flag mAb (Sigma). sCD160-Flag was finally eluted in 2mM glycine-HCl pH 2.8. The eluate was neutralized, submitted to dialysis in PBS, and concentrated (Centricon, Millipore, Bedford, MA). The protein concentration was then estimated on a silver-stained gel by comparison with known quantities of BSA.

### Immunoprecipitation and Immunoblotting

Culture medium (10 ml) from transfected COS7 cells was incubated with 5µg of anti-Flag M2 mAb for 1h30 at 4°C, and immune complexes were collected with 20µl of Protein G-Sepharose beads. Alternatively, 2x10⁷ control or IL-15-activated PB-NK lymphocytes were cultured for 24-48 h in 10 ml of RPMI 1640 medium without serum. Culture supernatants were collected and incubated with CL1-R2 mAb (10µg per test) followed by protein G-Sepharose beads. After washes, the precipitated proteins were separated by SDS-8% PAGE. The proteins were then transferred onto a nitrocellulose membrane and subjected to Western blot analysis using the anti-CD160 (CL1-R2, 5µg/ml) or anti-Flag M2 (5µg/ml) mAb. HRP-conjugated goat anti-mouse antibodies (Jackson Immunoresearch, Westgrove, PA) were used as secondary antibodies, and the immunoreactive proteins were visualized using an ECL kit (Amersham Biosciences).

### Lymphocyte mediated cytotoxicity.

The lymphocyte cytotoxicity was tested in a ⁵¹Cr-release assay. Target cells were labeled with 100 *µ*Ci of Na⁵¹CrO4 for 90 min at 37°C, and washed three times in RPMI 1640 medium containing 10% FCS. The target cells were then plated in 96-well V-bottomed microtiter plates (Greiner, Essen, Germany) for 1h at 37°C. When necessary, the cells were subjected to a pre-incubation step with 50µl of culture medium from COS7 cells transfected with pcDNA3 or sCD100-Flag expression vector. The effector cells were then added in a final volume of 150 µl per well. Assays at various E:T cell ratios on 10³ target cells were performed in triplicate. After 4 h of culture at 37°C, the plates were spun down and 100 µl of the cell supernatant were collected from each well. The determination of ⁵¹Cr release was done using a gamma-counter (Packard). The percentage of specific lysis was determined as previously reported (Maiza, H., G. Leca, I. G. Mansur, V. Schiavon, L. Boumsell, and A. Bensussan. 1993. J. Exp. Med. 178: 1121-1126). The lysis was considered as significant when representing more than 10% of the maximum level of cell lysis.

### Cell lines, peripheral blood cells and tissues

The HMC-1 mast cell line and the Cos, Jurkat and NK92 cell lines were cultured in RPMI 1640 supplemented with 10% fetal calf serum (FCS).

Human cord blood (CB-MC) and peripheral blood cytapheresis derived mast cells (C-MC) were obtained from CD34+ progenitors from healthy donors. Briefly, CD34+ progenitors from peripheral blood (cytapheresis) and cord blood were cultured in a mast cell culture medium, composed of α-minimal essential medium supplemented with FCS, bovine serum albumine, human recombinant stem cell factor and recombinant human IL-6. After more than 10 weeks in culture, more than 95% of the cells were identified as MCs according to their morphologic features.

Peripheral blood lymphocytes, natural killer cells (PB-NK cells) and basophils were isolated from peripheral blood of healthy donors. Briefly, peripheral blood mononuclear cells (PBMCs) were isolated from heparinized venous blood by density gradient centrifugation over lymphoprep (PAA Laboratories, Linz, Austria). PB-NK cells and basophils were purified by using the magnetic-activated cell sorter (MACS) NK cell isolation kit and basophil isolation kit, respectively (Miltenyi Biotec, Auburn, CA), following manufacturer's intructions.

The formalin fixed, paraffin embedded skin and salivary tissue samples, including skin biopsy from a patient with cutaneous mastocytosis (urticaria pigmentosa), were retrieved from the archival files of the department of Pathology of our institution (hôpital Henri Mondor, Créteil, France).

### RT-PCR amplification of β-actin and CD160 mRNAs

Total RNA was extracted from 1 to 5 x 10⁶ cells from cell lines and peripheral blood derived cells using the Trizol reagent (Invitrogen, Cergy-Pontoise, France) and chloroform/isopropanol precipitation. For reverse transcription, total mRNA (5-15 µg) was reverse transcribed by using oligo-dT primers and the Powerscript reverse transcriptase (RT Clontech, Palo Alto, CA).

PCR reactions were performed on 1 µg of total cDNA. Polymerase chain reaction (PCR) was performed using the following primers for CD160: forward (5'-TGCAGGATGCTGTTGGAACCC-3', SEQ ID n°1) and reverse (5'-CCTGTGCCCTGTTGCATTCTTC -3', SEQ ID n°2), flanking two 339 and 665 base pair segments from the two alternatively spliced short and long transcripts of CD160. RT-PCR for β-actin, used as a positive control, was done with previously reported primers, allowing amplification of a 245 base pair segment.

### Western Blotting

Culture supernatants and cell lysates from HMC-1 cells and supernatants from CB-MC and C-MC were collected and incubated with Tm60 mAb (10µg per test) followed by protein G-Sepharose beads. After washes, the precipitated proteins were separated by SDS-8% PAGE. The proteins were then transferred onto a nitrocellulose membrane and subjected to Western blot analysis using the anti-CD160 (CL1-R2) moAb, allowing detection of a specific 83 kD immunoprecipitate, or an isotypic control moAb. The anti-CD160 Tm60 moAb was raised and produced in our laboratory and used as purified ascites fluid. Horseradish peroxidase (HRP)-conjugated goat anti-mouse antibodies (Jackson Immunoresearch, Westgrove, USA) were used as secondary antibodies, and the immunoreactive proteins were visualized using a chemiluminescence kit (Amersham Biosciences).

### Immunohistochemistry

For immunostaining procedures on tissue samples, 3 micrometer-thick sections were applied on Superfrost plus slides (CML, Angers, France) and deparaffinized in xylene before use. For immunostaining procedure on HMC-1 cells, 2 x 10⁵ cells were applied to Superfrost plus slides by centrifugation using a Shandon cytospin 4 centrifuge (Thermo electron corporation, Waltham, MA), air dried and fixed in aceton. Primary moAbs to CD160 (CL1-R2) and mast cell tryptase (Dako SA, Glostrup, Denmark) were used at a 1:50 and 1:100 dilution, respectively. In tissue sections, moAbs were applied after rehydratation and antigen retrieval by heat in citrate buffer. The immunostaining procedure was performed using a biotin/avidine system conjugated to peroxydase (Vectastain ABC-P kit from Vector, Burlingame, USA). The peroxydase reaction was revealed by diaminobenzidine (Sigma-Aldrich, Saint Quentin Fallavier, France) and sections were counterstained in blue with hematoxylin. For immunofluorescent stainings, secondary biotinylated anti-mouse IgG antibodies and fluoroisothiocyanate conjugated streptavidine were used.

### Results

### CD160 membrane expression is decreased in IL-15 cultured PB-NK lymphocytes

Initial findings revealed a loss of BY55/CD160 cell surface expression on NK cells treated with PMA (Maiza, H., G. Leca, I. G. Mansur, V. Schiavon, L. Boumsell, and A. Bensussan. 1993. J. Exp. Med. 178: 1121-1126). More recently, we reported that NK lymphocytes cultured in the presence of IL-2 exhibit a decreased cell surface reactivity towards an anti-CD160 mAb, when compared to untreated cells (Le Bouteiller, P., A. Barakonyi, J. Giustiniani, F. Lenfant, A. Marie-Cardine, M. Aguerre-Girr, M. Rabot, I. Hilgert, F. Mami-Chouaib, J. Tabiasco, L. Boumsell, and A. Bensussan. 2002. Proc. Natl. Acad. Sci. USA 99: 16963-16968). Similar CD160 immunolabelings were performed on highly purified PB-NK cells consisting in CD56^{dim}CD160⁺ and CD56^{bright} CD160⁻ lymphocyte subsets in a 9/1 ratio (Fig. 1A). We observed that a short incubation time of these cells with IL-15 results in a strong decrease in anti-CD160 mAb recognition at their cell surface, as revealed by flow cytometry analysis (Fig. 1B). Both anti-CD160 antibodies, namely CL1-R2 and BY55, that are directed against distinct epitopes of the molecule, loss their reactivity towards IL-15-activated NK lymphocytes. Interestingly we found that, while CD160 molecules become undetectable at the cell surface of IL-15-activated CD56^{dim} NK lymphocytes, the level of CD160 transcripts is not modified in these cells and remains identical to the one detected in non treated cells (Fig. 1C, left panel). In contrast, and in agreement with their CD160⁻ phenotype, CD160 mRNA synthesis is not detected in resting CD56^{bright} NK cells, but is induced upon their incubation with IL-15 (Fig. 1C, right panel).

### Membrane-bound CD160 is cleaved through a metalloprotease-dependent process

The release of membrane-bound proteins under a soluble form, mediated through a proteolytic cleavage, has been reported for various molecules (McGeehan, G. M., J. D. Becherer, R. C. Bast, Jr., C. M. Boyer, B. Champion, et al. 1994. Nature. 370: 558-561 ; Salih, H. R., H. G. Rammensee, and A. Steinle. 2002. J. Immunol. 169: 4098-4102). We therefore investigated whether a similar mechanism can be responsible for the decreased detection of the GPI-anchored CD160 at the surface of activated CD56^{dim} NK lymphocytes. PB-NK lymphocytes were stimulated with IL-15 and further incubated in the presence of the phospholipase C (PLC)-type inhibitor U73122, or the GPI-specific phospholipase D (GPI-PLD) inhibitor 1,10-phenanthroline monohydrate (1,10 PNT). Cells were subsequently subjected to flow cytometry analysis to visualize membrane-bound CD160. The results shown in Fig. 2A demonstrated that the IL-15-induced down-modulation of CD160 cell surface expression is not affected by the addition of U73122 inhibitor. In contrast, it is partially impaired when 1,10 PNT is added to the cell culture medium. This first observation, together with previous studies reporting the involvement of the GPI-PLD1 protease in the release process of GPI-anchored membrane receptors ( Metz, C. N., G. Brunner, N. H. Choi-Muira, H. Nguyen, J. Gabrilove, I . W. Caras, N. Altszuler, D. B. Rifkin, E. L. Wilson, and M. A. Davitz. 1994. EMBO J. 13: 1741-1751; Naghibalhossaini, F., and P. Ebadi. P. 2006. Cancer Lett. 234: 158-167), prompted us to examine the expression of this enzyme in PB-NK lymphocytes. The presence of mRNA transcripts corresponding to the GPI-PLD1 known variant 1 and 2 (Schofield, J. N., and T. W. Rademacher. 2000. Biochim. Biophys. Acta. 1494: 189-194) was first assessed by RT-PCR. The results from a representative experiment performed on NK cells separated from the PBMC of a healthy individual indicate that neither the CD56^{bright}, nor the CD56^{dim}, PB-NK subsets express the transcripts for the GPI-PLD1 variant 2 (Fig. 2B). Note that the circulating PB-NK lymphocytes also show no synthesis of the GPI-PLD1 variant 1 transcript, while both variant 1 and 2 mRNAs are detected in PBMC and purified T lymphocytes (data not shown). Importantly, we established that the GPI-PLD1 variant 2 transcript synthesis is induced in both CD56^{bright} and CD56^{dim} NK pools when cultured in the presence of IL-15 (Fig. 2B). These data were further confirmed at the protein level by realizing immunostaining experiment with specific anti-PLD antibodies. Indeed, we observed an induction of the GPI-PLD protein expression in permeabilized circulating NK lymphocytes upon IL-15 treatment (Fig. 2C). Altogether these results demonstrate that the disappearance of CD160 from the NK cell membrane correlates with the neo-synthesis of the GPI-PLD enzyme, strongly suggesting that this Zn²⁺-dependent protease may be responsible for the cleavage of membrane-bound CD160 and its release under a soluble form.

### Characterization of soluble CD160 (sCD160) molecules released in IL-15-stimulated PB-NK cell extracellular environment

To definitely demonstrate the IL-15-mediated release of soluble CD160 by activated NK lymphocytes, and to better characterize this soluble form at the molecular level, anti-CD160 immunoprecipitates were prepared from resting or IL-15-treated PB-NK cell culture medium. The analysis of the immunoprecipitated proteins by Western blot using the anti-CD160 mAb CL1-R2 leads to the detection of a unique protein band with an apparent molecular mass of 80 kDa (Fig. 3). A similar recognition pattern was obtained when the anti-CD160 mAb BY55 was utilized for immunoprecipitation on YT and NK lymphocytes total cell lysates (data not shown), thus indicating that the membrane-bound and soluble forms of CD160 exhibit the same multimeric structure, which is resistant to reducing agents. Importantly, no protein band is immunoprecipitated from the culture supernatant of non-activated PB-NK lymphocytes, or of IL-15-stimulated cells cultured in the presence of 1,10-PNT inhibitor (Fig. 3). This latter observation indicates that sCD160 is not constitutively produced by circulating NK lymphocytes, and confirms the phospholipase-dependence of CD160 proteolytic cleavage.

### A sCD160-Flag fusion protein binds to MHC class I molecules and inhibits the activity of cytotoxic lymphocytes

To investigate the functional role of sCD160, we generated an expression vector coding for a C-terminal Flag-tagged soluble CD160 protein (sCD160-Flag). This fusion protein, when expressed by transiently transfected COS7 cells, exhibits the same multimeric structure that sCD160 molecules precipitated from an IL-15-treated PB-NK culture supernatant, as demonstrated by its detection as a 80 kDa polypeptide upon anti-CD160 mAb immunoblotting (Fig. 4A). By performing sCD160-Flag binding assays on HLA-Cw3-expressing 721.221 cells, we establish that sCD160-Flag protein efficiently interacts with the MHC-class I molecules, a maximum binding being observed at a concentration of 5µg/ml of recombinant protein (Fig. 4B). Importantly the use of a similar, or higher, concentration of sCD160-Flag fails to significantly label the parental cell line 221, inferring the specificity of the detected interaction.

The ability of sCD160 molecules to interact with MHC class I molecules led us to determine whether this association could functionally affect the MHC class I-restricted cytotoxic T lymphocyte (CTL) activities. Therefore, the cytolytic activity of the HLA-A11-restricted human cytotoxic T cell clone JF1 (David, V., J-F., Bourge, P. Guglielmi, D. Mathieu-Mahul, L. Degos, and A. Bensussan. 1987. J. Immunol. 138: 2831-2836) was tested against the specific HLA-A11 EBV-transformed B cell line. The target cells were pre-incubated with a culture supernatant obtained from COS7 cells transfected with either the empty expression vector (control) or sCD160-Flag coding construct. A representative experiment, shown in Fig. 4C (left panel), reveals that sCD160 partially inhibits the specific CTL activity exerted by the JF1 clone. The level of inhibition observed never exceeded 25-30% for all effector/target cell ratios tested. Furthermore, the incubation of the target cells with higher concentration of sCD160-Flag (> 5µg/ml) did not result in the detection of higher inhibition levels, and almost no inhibition was obtained when less than 1µg/ml of sCD160-Flag was used (data not shown). Importantly, a sCD160-induced inhibition of cytotoxicity is also observed when CD8⁺ CTL isolated from 6-days allogeneic mixed lymphocyte cultures are used as effector cells (Fig. 4C, right panel). This suggests that the sCD160-mediated down-modulation of cytolytic activity is not restricted to cytotoxic T cell clones, but could also be effective on allogeneic stimulated T lymphocytes.

We previously reported that the lysis of K562 cells by freshly isolated PB-NK lymphocytes (that expressed significant amount of CD160, see Fig. 1B) is partly dependent on CD160/HLA-C interaction (Barakonyi, A., M. Rabot, A. Marie-Cardine, M. Aguerre-Girr, B. Polgar, V. Schiavon, A. Bensussan, and P. Le Bouteiller . 2004. J. Immunol. 173: 5349-5354). In addition, the K562 NK lymphocyte-sensitive target cells expressed low amounts of MHC class I molecules, as demonstrated by flow cytometry analysis using the anti-MHC-I mAb W6/32 (Fig. 5A, left panel). Note that we recently identified the MHC-I molecules expressed by these NK cell targets as HLA-Cw3 (Barakonyi, A., M. Rabot, A. Marie-Cardine, M. Aguerre-Girr, B. Polgar, V. Schiavon, A. Bensussan, and P. Le Bouteiller . 2004. J. Immunol. 173: 5349-5354). We further establish that sCD160-Flag protein binds to the HLA-C molecules expressed by K562 cells (Fig. 5A, right panel). Consequently, a significant inhibition of PB-NK lymphocyte cytotoxicity towards K562 cells is observed (Fig. 5B). As reported elsewhere, the addition of the anti-CD160 mAb CL1-R2 (Fig. 5B), or of the anti-MHC-I mAb W6/32 (data not shown), similarly impairs the PB-NK cell-mediated cytotoxicity against K562 cells. These data indicate that the binding of sCD160 to MHC class I molecules impairs the recognition of the target cells by the cytotoxic effector cells, thus decreasing their cytolytic activity.

### Characterization of soluble CD160 (sCD160) molecules released by T cells or by mast cells.

Nikolova et al. showed that CD160 cellular membrane expression is dowmodulated in lymphocytes activated for a few hours (Nikolova et al. Int Immunol. 2002 May;14(5):445-51). Culture supernatants of these activated cells were thus assessed for the presence of soluble CD160, and soluble CD160 was detected, demonstrating that activated T cells are capable of producing soluble CD160 (data not shown).

The capacity of mast cells to express CD160 and produce soluble CD160 was also assessed (figure 6).

### RT PCR for CD160 in HMC-1 and peripheral Basophils from a healthy donor

A RT-PCR was done using standard procedure and specific primers for β actin and CD160. mRNA from cell lines and peripheral leukocytes were purified using the Trizol ragent technique. HMC-1 cells (a mast cell line) as well as peripheral basophils from an healthy donor display CD160 mRNA expression, with the two alternatively spliced short and long transcripts of 339 and 665 base pair, respectively. A similar expression is found in PBL and NK cells from healthy donors and in NK92 cells. As negative controls, Cos and Jurkat cells do not show CD160 expression.

### CD160 immunoprecipitation in culture supernatant of human mast cells

CD160 immunoprecipitation was done in HMC-1 cells lysate and supernatant, as well as in supernatant of human mast cells grown in culture, from CD34⁺ pluripotent progenitor derived from cord blood (CB-MC) or cytapheresis (C-MC) of healthy donors. Following protein separation, immunoblotting was performed using the Tm60 monoclonal antibody (CL1-R2) and a horseradish peroxidase-conjugated secondary antibody. A specific 83 kD immunoprecipitate is found in the supernatant of both HMC-1 cells and CD34+ progenitor cells derived human mast cells, indicating that these cells produce soluble CD160.

### Immunohistochemical expression of CD160 by human mast cells from normal tissues and cutaneous mastocytosis and HMC-1

Standard immunohistochemical detection of CD160 and mast cell tryptase was done with the avidin biotin-peroxydase technique, using Tm60 monoclonal antibody (CL1-R2) (A, C, E) and (B, D, F). For immunofluorescent tests, secondary biotinylated anti-mouse IgG antibodies and fluoroisothiocyanate conjugated streptavidine were used (G, H). In normal skin and accessory salivary gland paraffin embedded tissue sections, mast cells are strongly stained with anti-CD160 monoclonal antibodies (A, C, arrows). In the skin, they are located in the papillary dermis and around dermal capillaries of the mid and deep dermis (A). In salivary gland, they are seen around the salivary ducts and acini (C). In both skin and salivary gland, mast cells are characterized morphologically and by mast cell tryptase expression (B, D). HMC-1 cells strongly express CD160, especially within cytoplasmic granules (E), as well as mast cell tryptase (F). Using immunofluorescent tests, mast cells from cutaneous mastocytosis (G) and HMC-1 cells (H) display strong granular expression of CD160 (arrows).
(Magnifications : A,B,C,D,E,F X200 ; G, H : X400)

## Claims

1. Pharmaceutical composition comprising a soluble form of CD160.

2. Pharmaceutical composition according to claim 1, further comprising at least one acceptable carrier.

3. Pharmaceutical composition according to any one of the claims 1 to 2, suitable for oral, rectal, nasal, topical or parenteral administration, preferably for parenteral administration.

4. Pharmaceutical composition according to any one of the claims 1 to 3, for treating an inflammatory condition involving an undesired immune response, such as tissue graft or organ rejection, and autoimmune diseases.

5. Pharmaceutical composition according to claim 4, for treating organ rejection such as heart, kidney and liver rejection.

6. Pharmaceutical composition according to claim 4, for treating inflammatory diseases such as rheumatoid arthritis, atopic dermatitis, psoriasis, multiple sclerosis, diabetes, lupus and inflammatory bowel diseases.

7. The use of a soluble form of CD160 for the preparation of a pharmaceutical composition for treating inflammatory conditions such as tissue graft or organ rejection and autoimmune diseases.

8. The use according to claim 7 for treating organ rejection such as heart, kidney and liver rejection.

9. The use according to claim 7 for treating inflammatory diseases such as rheumatoid arthritis, atopic dermatitis, psoriasis, multiple sclerosis, diabetes, lupus and inflammatory bowel diseases.

10. The use according to any one of the claims 7 to 9, in combination with at least one immunosuppressive agent for treating inflammatory conditions.

11. The use according to claim 10, wherein said immunosuppressive agent is cyclosporine A, FK506, rapamycin, steroids such as glucocorticosteroid (most preferably prednisone or methylprednisolone), cytostatics such as methotrexate, azathioprine, and monoclonal antibodies such as OKT3 or anti-TNF.

12. Kit for treating an inflammatory condition such as tissue graft or organ rejection and autoimmune diseases comprising a pharmaceutical composition according to any one of the claims 1 to 6 and at least one immunosuppressive agent.

13. An in vitro method for screening an individual for the presence of an inflammatory condition such as infectious and autoimmune diseases, tissue graft and organ rejection, or the presence of a tumor or activated endothelial cells, wherein a soluble CD160 is used as a marker.

14. An in vitro method for monitoring therapy of an inflammatory condition such as an autoimmune disorder or a tissue or organ rejection or for monitoring the presence of a tumor during chemotherapy including treatment with an anti-angiogenic substance or antibody, wherein a soluble CD160 is used as a marker.

15. The method according to claim 13, comprising detecting the level of soluble CD160 in a biological sample from said individual and comparing the level of soluble CD160 in said sample to the level of soluble CD160 from a control population, wherein an increase in the level of soluble CD160 is indicative of an inflammatory condition such as infectious and autoimmune condition, of a tissue graft or organ rejection or of the presence of a tumor or activated endothelial cells in said individual.

16. The method according to claim 14, comprising detecting the level of soluble CD160 in a biological sample from an individual undergoing treatment for said inflammatory conditions or chemotherapy including treatment with an anti-angiogenic substance or antibody and comparing the level of soluble CD160 in said sample to a baseline level of soluble CD160 present in said individual, wherein a decrease in the level of soluble CD160 relative to the baseline level is indicative of a positive response to treatment.

17. The method according to any one of the claims 13 to 16, wherein said organ rejection comprises heart, kidney or liver rejection.

18. The method according to any one of the claims 13 to 16, wherein said inflammatory diseases comprise rheumatoid arthritis, atopic dermatitis, psoriasis, multiple sclerosis, diabetes, lupus and inflammatory bowel diseases.

19. The method according to any one of the claims 13 to 18, wherein said biological sample comprises serum, plasma, urine, cerebrospinal fluid, joint fluid, ascites or amniotic fluid.

20. The method according to any one of the claims 13 to 19, comprising:
- contacting a biological sample with a ligand that binds to soluble CD160, and
- detecting the binding of soluble CD160 to said ligand.

21. The method according to claim 20, wherein said ligand comprises an antibody that binds to soluble CD160 or one of CD160 receptors such as classical or non classical MHC class I molecules or CD1 molecules.

22. The method according to any one of the claims 13 to 21, wherein the level of soluble CD160 is detected using a monoclonal antibody.

23. The method according to any one of the claims 13 to 22, wherein the level of soluble CD160 is detected using a capture antibody and a detection antibody, wherein said detection antibody comprises a label.

24. The method according to any one of the claims 13 to 23, wherein said capture antibody is attached to a solid substrate.

25. The method according to claim 24, wherein said solid substrate comprises a bead or a microtiter plate.

26. Kit comprising:
- at least one ligand having a specific binding affinity for soluble CD160, said ligand comprising an antibody or a classical or non classical MHC class I molecule or a CD1 molecule, and
- reagents such as secondary antibodies.
